# EUROPÄISCHE PATENTSCHRIFT

(11) **EP 0 860 148 B1**
(45) Veröffentlichungstag und Bekanntmachung des Hinweises auf die Patenterteilung: **01.06.2005**
(21) Anmeldenummer: 98102985.3
(22) Anmeldetag: 20.02.1998
(51) Int. Cl.: A61B 17/28, F16B 7/20, F16L 37/252

(54) **Bajonettkupplung zum lösbaren Verbinden zweier Rohrschaftinstrumente oder -instrumententeile**
Bayonet coupling for the releasable connection of two tubular-shaft instruments or two parts of an instrument
Attache de type à baionnette pour l'assemblage démontable de deux instruments tubulaires ou de deux pièces d'un instrument

(30) Priorität: 25.02.1997 DE 19707373
(43) Veröffentlichungstag der Anmeldung: 26.08.1998
(73) Patentinhaber: Karl Storz GmbH & Co. KG, 78532 Tuttlingen (DE)
(72) Erfinder: Dittrich, Horst, 78194 Immendingen (DE); Doll, Frank, 78589 Dürbhein (DE)
(74) Vertreter: Weller, Wolfgang, Dr.rer.nat.

(56) Entgegenhaltungen:
- EP-A- 0 705 571
- DE-A- 4 307 539
- DE-A- 4 323 093
- DE-U- 29 613 701
- US-A- 5 582 615

## Beschreibung

Die Erfindung betrifft eine Bajonettkupplung zum lösbaren Verbinden zweier Rohrschaftinstrumente oder -instrumententeile, mit einer eine Bajonettführung aufweisenden Bajonetthülse (weibliches Teil) an dem einen Instrument bzw. Instrumententeil und einem Bajonetteinsatz (männliches Teil) am anderen Instrument bzw. Instrumententeil, und mit einer Verriegelung zum Verriegeln der geschlossenen Bajonettkupplung, wobei die Verriegelung ein längs der Bajonetthülse verschiebbares Verriegelungsstück aufweist, das mit dem in die Bajonetthülse eingesetzten Bajonetteinsatz in dessen Endstellung unmittelbar in sperrenden Eingriff kommt und den Bajonetteinsatz gegen Verdrehen sperrt, und das, zum Lösen der Verriegelung, vom Bajonetteinsatz weg bewegbar ist.

Eine derartige Bajonettkupplung ist aus der EP 0 705 571 A bekannt.

Bei dieser Bajonettkupplung weist die Bajonetthülse eine Bajonettführung auf, die einen zunächst in axialer Richtung verlaufenden Abschnitt aufweist, der in einen umfänglichen Abschnitt übergeht, an dessen äußerem Ende ein wieder in axialer Richtung zurücklaufender Abschnitt vorgesehen ist. Beim Einschieben des Bajonetteinsatzes, also des männlichen Teils, wird ein von diesem radial vorspringender Zapfen in die Bajonettführung eingeschoben. Der Zapfen wird soweit axial vorgeschoben, bis er den umfänglichen Abschnitt erreicht, in dieser Stellung kann dann das eingeschobene männliche Teil relativ zum weiblichen Teil verdreht werden. Da das Verriegelungsstück aufgrund einer Feder so vorgespannt ist, daß diese sich auf den Bajonetteinsatz hin bewegt, wird, nachdem der Bajonetteinsatz in seiner Enddrehstellung unmittelbar in sperrendem Griff mit dem Verriegelungsstück gekommen ist, dieser Zusammenbau in den axial rückspringenden Abschnitt der Bajonettführung zurückgeschoben. Dadurch ist dann die geschlossene Kupplung in der Endstellung gegen Verdrehen gesperrt.

Zum Lösen der Kupplung muß der Zusammenbau aus Bajonetteinsatz und Verriegelungsstück gegen die Kraft der Feder axial bewegt werden, und zwar soweit, bis der vom Bajonetteinsatz radial vorspringende Zapfen in den umfänglichen Abschnitt der Bajonettführung eintritt, dann muß dieser Zusammenbau gedreht werden und kann dann über den offenen Abschnitt der Bajonettführung axial abgezogen werden.

Anders ausgedrückt, muß der Zusammenbau aus Bajonetteinsatz und Verriegelungsstück etwas in der Richtung, in der die Kupplung geschlossen wird, gegen die Kraft der Feder verschoben werden, anschließend gedreht werden und kann dann gelöst werden.

Aus der US-A-5 407 293 ist eine Kupplung für ein medizinisches Instrument bekannt, bei der im weiblichen Teil zwei axial verlaufende Nuten eingeschnitten sind, in die entsprechende äußere Vorsprünge am männlichen Teil eingeschoben werden können. Ferner ist noch eine umfängliche Nut im Abstand von einer äußeren Stirnseite des weiblichen Teils eingeschnitten, durch die die beiden längsverlaufenden Nuten hindurchreichen. Im Innern der Hülse, also des weiblichen Teils, ist ein axial verschiebbares Verriegelungselement angeordnet, das gegen die Kraft einer Feder hin- und herbeweglich ist. Dieses Verriegelungselement weist zwei Arme auf, die in dem längsverlaufenden Nuten des weiblichen Teils angeordnet sind und diese ausfüllen.

Zum Schließen der Kupplung muß das Verriegelungsstück etwas gegen die Kraft der Feder zurückgeschoben werden, dann kann das männliche Teil soweit in die axialen Nuten eingesteckt werden, bis diese auf Höhe der umfänglichen Nut liegen. Dann wird die Kupplung verdreht, dabei werden die seitlichen Vorsprünge am männlichen Teil aus der Orientierung mit den längsverlaufenden Nuten herausgedreht und in die umfängliche Nut hineinbewegt.

Anschließend wird das Verriegelungselement losgelassen, die Arme fahren axial in den Nuten vor und sperren somit ein Verdrehen des männlichen Teils.

Zum Lösen der Kupplung muß dieses Verriegelungselement zurückgezogen werden, erst dann kann das männliche Teil wieder relativ zum weiblichen Teil verdreht und abgezogen werden.

In der Medizintechnik besteht ein zunehmender Bedarf an Instrumenten und insbesondere an Rohrschaftinstrumenten, die durch wenige Handgriffe zerlegbar sind, um die Instrumente nach einer Operation zu reinigen und zu sterilisieren. Ferner haben in der Medizintechnik Modulbauweisen Eingang gefunden, bspw. können an Rohrschaftinstrumenten mit scherenartigen Handgriffen am distalen Ende unterschiedliche Instrumententeile wie Schneiden, Faßzangen oder dgl. angebracht werden.

In der minimalinvasiven Chirurgie ist es häufig erforderlich, ein Endoskop, einen Trokar, einen Zange, eine Schere oder ein anderes Instrument in einen Schaft einzusetzen, der bspw. ein Universalschaft, eine Trokarhülse oder dgl. sein kann. Es ist dabei erforderlich, daß das mit dem Schaft verbundene Instrument im Einsatz fest mit dem Schaft verbunden ist, und bei Bedarf wieder einfach gelöst werden kann.

Zur Herstellung dieser festen Verbindung ist eine Kupplung vorgesehen. In der Regel sind diese Kupplungen als Bajonettkupplungen ausgebildet, d.h. an einem der Rohrschaftinstrumente bzw. -instrumententeile ist eine eine Bajonettführung aufweisende Bajonetthülse (weibliches Teil) vorgesehen, in das ein Bajonetteinsatz (männliches Teil) eingeschoben bzw. eingedreht werden kann.

Damit bei der Handhabung durch Drehen der verkuppelten Teile relativ zueinander die Bajonettkupplung nicht wieder gelöst wird, sind Verriegelungen vorgesehen.

Bei der DE 43 07 539 A1 ist die Bajonetthülse am distalen Ende eines Rohrschaftes einer Zange vorgesehen. Der Bajonetteinsatz ist am distalen Endbereich eines stangenförmigen Betätigungselementes vorgesehen, das am distalen Ende die Zangenmaulteile trägt. Das proximale Ende des stangenförmigen Betätigungselementes dient zur Verbindung mit einem beweglichen Griffelement des scherenartigen bzw. anderweitig angeführten Handgriffes. Beim Verkuppeln dieser beiden Teile wird das proximale Ende des stangenförmigen Betätigungselementes distal in den Schaft ein- und durch diesen hindurchgeschoben bis der Bajonetteinsatz mit der Bajonetthülse in Eingriff kommt. Nachdem der Bajonetteinsatz in die Bajonettführung der Bajonetthülse eingeschoben und verdreht wurde, werden die beiden Bauelemente in dieser Stellung miteinander verriegelt. Dazu sind am proximalen Ende des stangenförmigen Betätigungselements zwei sich axial erstreckende Abflachungen vorgesehen, in die radial Klemmelemente eingelegt werden, die über einen Federring in der klemmenden Stellung gehalten werden. Die Klemmelemente verhindern eine Relativdrehung zwischen Schaft und stangenförmigem Betätigungselement.

Diese Verriegelung ist umständlich zu handhaben und erfordert maßgenaues Bearbeiten der Zugstange und der Hülse am proximalen Ende.

Aufgrund der Tatsache, daß die eigentliche Bajonettkupplung am distalen Ende angeordnet ist, die Verriegelung aber am proximalen Ende des Rohrschaftinstrumentes, können auf das Rohrschaftinstrument einwirkende Drehmomente dennoch ein Lösen der Kupplung verursachen. Das stangenförmige Betätigungselement weist bei üblichen Zangen Durchmesser im Bereich von einigen Millimetern jedoch Längen im Bereich von 20 cm und mehr auf. Das heißt, die Kupplung befindet sich etwa in einem axialen Abstand von 20 cm zur Verriegelung. Drehmomente können nunmehr eine solche Torsion des langen und dünnen stangenförmigen Betätigungselementes verursachen, daß das distale Ende der Stange soweit relativ zum verriegelten proximalen Ende verdreht wird, daß sich die Kupplung löst.

Solche Drehmomente wirken auf Instrumente ein, wenn bspw. mit dem am distalen Ende arbeitenden Instrumententeil ein Gewebeteil, bspw. Knorpel, erfaßt und durch Schließen und Drehen des Instrumentes das erfaßte Gewebestück abgetrennt werden soll.

Aus der DE 43 23 093 A1 ist eine ähnliche chirurgische Zange bekannt, bei der ebenfalls am distalen Ende die Bajonettkupplung vorgesehen ist, am proximalen Ende die Verriegelung. Die Verriegelung wird so bewerkstelligt, daß am proximalen Ende des stangenförmigen Betätigungselementes ein sich axial erstreckendes Federelement vorgesehen ist, das in einen Längsschlitz am proximalen Ende des Schaftes einrastet. Auch hier kann eine Torsion des stangenförmigen Betätigungselementes dazu führen, daß die Kupplung gelöst wird.

Ein solches Lösen kann für den Patienten fatale Folgen haben, denn das Instrument kann dann nicht mehr betätigt werden und löst sich im Körper des Patienten in zwei Teile auf.

Es ist daher Aufgabe der vorliegenden Erfindung, eine Bajonettkupplung der eingangs genannten Art dahingehend weiterzuentwickeln, daß die Verriegelung sicher ist, insbesondere hohe Drehmomente, die auf die Bajonettverbindung einwirken, aufnehmen kann.

Erfindungsgemäß wird die Aufgabe dadurch gelöst, daß das Verriegelungsstück mit einer äußeren Schiebehülse verbunden ist, die von Hand ergreifbar und verschiebbar ist.

Entkupplungen durch Torsionsbewegungen oder durch Druckbewegungen von Bauelementen zwischen Bajonettkupplung und Verriegelung sind grundsätzlich ausgeschlossen. Aufgrund der Tatsache, daß die Verriegelung direkt, also unmittelbar mit dem Bajonetteinsatz in sperrendem Eingriff kommt, können sehr hohe Drehmomente aufgenommen werden. Dadurch, daß das Verriegelungsstück lediglich axial, also längs der Bajonetthülse verschiebbar ist und mit dem Bajonetteinsatz in dessen Enddrehstellung in sperrenden Eingriff kommt, ist ausgeschlossen, daß sich der Bajonetteinsatz aus dessen Enddrehstellung herausdrehen kann. Dies wäre aber Voraussetzung, daß sich die Bajonettverbindung löst, d.h. der Bajonetteinsatz muß zunächst um einen gewissen Betrag verdreht werden, was nunmehr aber wirksam gesperrt ist.

Darüber hinaus ist das Schließen und das Lösen der Kupplung einfach dadurch zu bewerkstelligen, daß das Verriegelungsstück axial längs der Bajonetthülse verschoben wird, entweder in Richtung der sperrenden Stellung zum Verriegeln, oder in die entgegengesetzte Richtung zum Lösen der Verriegelung. Dazu wird das Verriegelungsstück mittels der von außen zugänglichen Schiebehülse bewegt. Die Bajonettkupplung samt Verriegelung ist ein kompaktes Bauelement, d.h. alle Bauelemente sind auf den Bereich der Bajonettkupplung beschränkt. Dies eröffnet auch eine sehr flexible Anordnung der Bajonettkupplung, sei es am distalen oder am proximalen Ende eines Rohrschaftinstrumentes oder auch dazwischenliegend je nach Belieben.

Die Kupplung ist nun auch unabhängig davon, ob ein stangenförmiges Betätigungselement wie eingangs beschrieben vorhanden ist oder nicht. Die nunmehr vorgeschlagene Kupplung arbeitet unabhängig von Bauelementen anderer Funktion, ist somit wesentlich universeller und sicherer einsetzbar.

Somit wird die Aufgabe vollkommen gelöst.

In einer weiteren Ausgestaltung der Erfindung ist das Verriegelungsstück durch die Kraft einer Feder beaufschlagt, die das Verriegelungsstück in den sperrenden Eingriff mit dem Bajonetteinsatz drückt.

Diese Maßnahme hat nun den Vorteil, daß das Verriegelungsstück dauernd in die sperrende Stellung gedrückt wird, somit eine geschlossene Kupplung sicher sperrend verriegelt ist. Zum Lösen der Kupplung muß das Verriegelungsstück gegen die Kraft der Feder zunächst vom Bajonetteinsatz abgezogen werden, erst dann kann dieser verdreht werden. Dies ist durch eine einfach zu handhabbare Verschiebebewegung längs des Rohrschaftinstrumentes möglich, ohne daß andere Manipulationen notwendig sind, wie bspw. Zerlegen des Schaftes oder Zerlegen von Griffelementen.

In einer weiteren Ausgestaltung der Erfindung weist das Verriegelungsstück auf der dem Bajonetteinsatz zugewandten Seite eine Ausnehmung auf, die, in der Endstellung des Bajonetteinsatzes, auf einen Stirnabschnitt des Bajonetteinsatzes aufschiebbar ist, und das aufgeschobene Verriegelungsstück sperrt ein Drehen des Bajonetteinsatzes.

Diese Maßnahme hat den Vorteil, daß eine kompakt bauende Verriegelung möglich ist, die auch bei dünnen Rohrschaftinstrumenten eine ausreichende Verdrehsicherung bietet, d.h. also hohe Drehmomente aufnehmen kann.

In einer weiteren Ausgestaltung der Erfindung weist das Verriegelungsstück eine solche, dem Bajonetteinsatz zugewandte Stirnfläche auf, daß der in die Bajonetthülse eingeschobene Bajonetteinsatz das Verriegelungsstück zunächst zurückschiebt und bis in die Endstellung relativ zum Verriegelungsstück verdrehbar ist.

Diese Maßnahme hat nun den Vorteil, daß die Steuerung bzw. das Verschieben des Verriegelungsstückes vom Bajonetteinsatz selbst durchgeführt wird, ohne daß dazu weitere konstruktive Bauelemente notwendig sind. Der Bajonetteinsatz schiebt das Verriegelungsstück, nachdem er auf dessen Stirnfläche getroffen ist, zurück und kann relativ zum Verriegelungsstück verdreht werden, bis das Verriegelungsstück axial auf den Bajonetteinsatz aufschiebbar ist, was bei der Ausgestaltung mit dem federbelasteten Verriegelungsstück dann automatisch erfolgt. Das bedeutet, die Handhabungsperson braucht zum Verriegeln keine andere Bewegungen durchzuführen als zum Bewerkstelligen der Bajonettkupplung, also Einschieben und Verdrehen, dabei wird dann automatisch am Ende der Drehbewegung der Bajonetteinsatz verriegelt. Zum Lösen braucht dementsprechend nur noch das Verriegelungsstück, in der Ausgestaltung mit der äußeren Schiebehülse, von Hand verschoben werden, um die Kupplung wieder zu lösen.

In einer weiteren Ausgestaltung der Erfindung weist das Verriegelungsstück eine mittig durchgehende Bohrung auf.

Diese Maßnahme hat den Vorteil, daß durch das Verriegelungsstück hindurch Bauelemente, wie bspw. ein stangenförmiges Betätigungselement hindurchreichen kann, so daß dann die Bajonettkupplung an irgendeiner bestimmten axialen Stelle eines Rohrschaftinstrumentes mit einem solchen Betätigungselement angeordnet werden kann.

Dazu sind dann alle Bauteile mit einer durchgehenden Bohrung bzw. Öffnung versehen.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachfolgend anhand eines ausgewählten Ausführungsbeispiels näher beschrieben und erläutert. Es zeigen:
- Fig. 1: in einer Explosionsdarstellung die wesentlichen Bauelemente einer erfindungsgemäßen Bajonettkupplung, und
- Fig. 2: einen Längsschnitt der beiden Bauelemente in montiertem, jedoch noch nicht verkuppelten Zustand.

Eine in den Figuren mit der Bezugsziffer 10 versehen Bajonettkupplung weist eine Bajonetthülse 12 auf.

In der Bajonetthülse 12 ist eine Bajonettführung 14 vorgesehen, die diametral gegenüberliegend jeweils einen von einem Einschubende 15 ausgehenden Längsschlitz 16 aufweist, der an dessen hinterem geschlossenen Ende in einen Querschlitz 18 übergeht, wie das bei Bajonettführungen üblich ist.

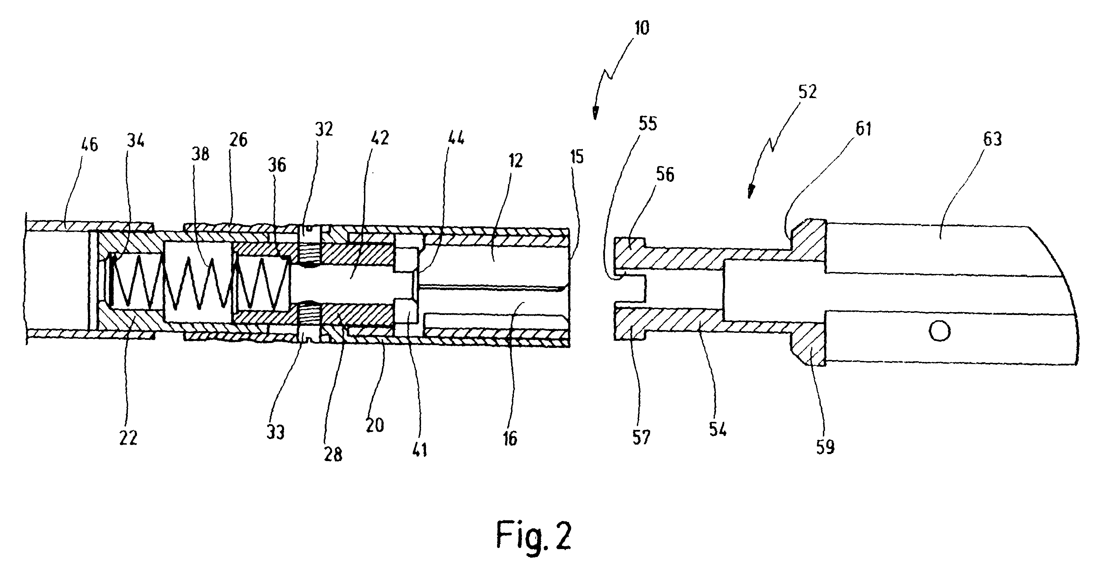

Die Bajonetthülse 12 ist in einer Hülse 20 aufgenommen und drehfest mit dieser verbunden (siehe Schnittdarstellung von Fig. 2).

Die Hülse 20 setzt sich in einem etwas durchmessergeringeren Rohrstück 22 fort, an dessen Außenseite diametral gegenüberliegend zwei Längsschlitze 24 vorgesehen sind.

Auf das durchmessergeringere Rohrstück 22 kann eine Schiebehülse 26 aufgeschoben werden, deren lichter Innendurchmesser etwa dem Außendurchmesser des Rohrstückes 22 entspricht. In der Schiebehülse 26 sind diametral gegenüberliegende Löcher 27 bzw. 27' vorgesehen, die mit den Längsschlitzen 24 fluchten. Im Innern der Hülse 20 ist ferner ein hohlzylindrisches Verriegelungsstück 28 aufgenommen, das ebenfalls diametral gegenüberliegende Gewindebohrungen 30 aufweist, die mit den Längsschlitzen 24 des Rohrstückes 22 bzw. mit den Löchern 27, 27' der Schiebehülse 26 fluchten.

Im montierten Zustand, siehe Fig. 2, ist das Verriegelungsstück 28 in das Rohrstück 22 eingeschoben und auf dessen Außenseite ist die Schiebehülse 26 aufgeschoben, wobei dies so erfolgt, daß die Bohrungen 30 im Verriegelungsstück mit den Längsschlitzen 24 bzw. den Löchern 27, 27' in der Schiebehülse 26 fluchten. Durch Eindrehen von Schrauben 32 bzw. 33 ist die Schiebehülse 26 fest mit dem Verriegelungsstück 28 verbunden.

Der Zusammenbau aus Schiebehülse 26 und Verriegelungsstück 28 ist jedoch axial, entsprechend der Länge der Längsschlitze 24, hin- und herverschiebbar, wie das durch den Doppelpfeil 47 angedeutet ist.

Wie aus der Schnittdarstellung von Fig. 2 zu entnehmen, ist im Innern des Rohrstückes 22 ein sacklochartiges Widerlager 34 vorgesehen, dem ein entsprechendes Widerlager 36 im Verriegelungsstück 28 gegenüberliegt.

Diese Widerlager 34 und 36 dienen als Abstützstellen für eine Schraubenfeder 38, die im montierten Zustand (Fig. 2) auf Druck vorgespannt ist.

Aufgrund der Druckkraft der Feder 38 wird somit der Zusammenbau aus Schiebehülse 26 und Verriegelungsstück 28 in Richtung Einschubende der Bajonetthülse 12 gedrückt.

An dem dem Einschubende der Bajonetthülse 12 zugewandten Ende weist das Verriegelungsstück 28 eine Ausnehmung 40 auf. Die Ausnehmung 40 entspricht einem radialen mittigen Einschnitt, so daß beidseits der Ausnehmung 40 sich axial erstreckende Vorsprünge 41 vorhanden sind.

Der Bereich der Ausnehmung 40 bzw. die diese flankierenden Vorsprünge 41 liegt im Bereich des Querschlitzes 18 der Bajonettführung 14, wie das insbesondere aus der Schnittdarstellung von Fig. 2 ersichtlich ist.

Das Verriegelungsstück 28 weist auf diesem, dem Einschubende der Bajonetthülse 12 zugewandten Ende eine ebene glatte Stirnseite 44 auf.

Der zuvor erwähnte Zusammenbau ist am distalen Ende eines Schaftes 46 eines Rohrschaftinstrumentes vorgesehen, bspw. einer medizinischen Zange.

Die Bajonettkupplung 10 weist ferner einen Bajonetteinsatz 52 auf.

Der Bajonetteinsatz 52 weist einen hohlzylindrischen Abschnitt 54 auf, in dessen Stirnabschnitt 55 radial über den hohlzylindrischen Abschnitt vorstehende Zapfen 56 und 57 vorgesehen sind.

Die Kontur der Zapfen 56 und 57 ist so gewählt, daß diese in die Bajonettführung passend eingeführt werden können. An dem den Zapfen 56 und 57 gegenüberliegenden Ende ist der hohlzylindrische Abschnitt 54 mit einer Schulter 59 versehen, die einen Anschlag 61 bildet.

Der hohlzylindrische Abschnitt 54 erstreckt sich in einem Montageansatz 63 weiter. Auf den Montageansatz ist ein distales Arbeitselement montiert, in dem zuvor erwähnten Beispiel einer medizinischen Zange sind dies entsprechende Zangenmaulteile.

Aus der Schnittdarstellung von Fig. 2 ist zu erkennen, daß beide Bauelemente der Bajonettkupplung 10 eine mittige durchgehende Öffnung aufweisen, durch die bspw. ein stangenförmiges Betätigungselement zum Betätigen der auf dem Montageansatz 63 montierten Zangenmaulteile hindurchgeschoben werden kann.

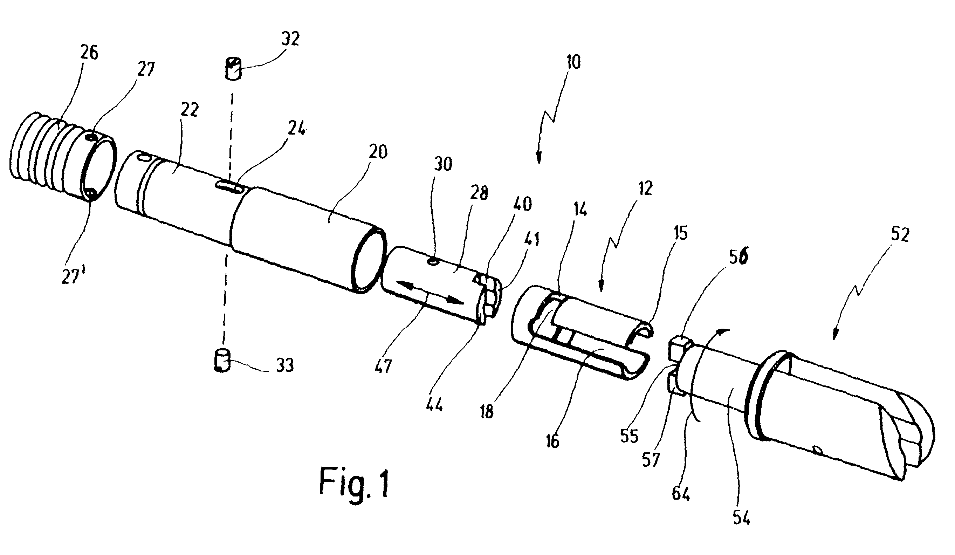

Zum Schließen der Bajonettkupplung 10 wird der Bajonetteinsatz 52 gegenüber der Darstellung von Fig. 1 und 2 um 90° verdreht, und so an der Bajonetthülse 12 angesetzt, daß die Zapfen 56 und 57 in die diametral gegenüberliegenden Längsschlitze 16 der Bajonettführung 14 eingreifen können. Der Bajonetteinsatz 52 wird soweit vorgeschoben, bis dessen Stirnseite auf die Stirnseite 44 des Verriegelungsstückes 28 trifft. Ein weiteres Einschieben verschiebt das Verriegelungsstück 28 gegen die Kraft der Feder 38 soweit, bis die Zapfen 56 bzw. 57 vor den Querschlitz 18 kommen. Nunmehr kann der eingeschobene Bajonetteinsatz 52 verdreht werden (siehe Pfeil 64), wobei die Zapfen 56 und 57 in die Querschlitze 18 eintreten. Hat der Bajonetteinsatz 52 seine Enddrehstellung erreicht, daß ist die in den Fig. 1 und 2 gezeigte Drehstellung, kommen dessen Zapfen 56 und 57 direkt vor der Ausnehmung 40 in der Stirnseite 44 des Verriegelungsstückes 28 zum Liegen.

Die Feder 38 schiebt nun das Verriegelungsstück 28 über die Zapfen 56 bzw. 57, wobei diese dann in die Ausnehmung 40 eintreten, die dann dementsprechend geformt ist, d.h. daß die Zapfen 56 und 57 passend eintreten können.

In diesem sperrenden Eingriff zwischen Verriegelungsstück 28 und Bajonetteinsatz 52 ist ein Drehen des Bajonetteinsatzes 52 in der Bajonetthülse 12 gesperrt. Die Kraft der Druckfeder 38 ist so eingestellt, daß bei den üblichen Handhabungen auf die Verschiebehülse 26 einwirkende axiale Kräfte nicht dazu ausreichen, die Schiebehülse 26 axial zu verschieben.

Zum Lösen der Bajonettkupplung 10 muß lediglich die Schiebehülse 26 gegen die Kraft der Feder 38 verschoben werden, wobei das Verriegelungsstück 28 von den Zapfen 56 und 57 des Bajonetteinsatzes 52 abgezogen werden, so daß dieser dann verdreht werden kann.

Schon nach einem geringfügigen Verdrehen des Bajonetteinsatzes 52 kann die Schiebehülse 26 wieder losgelassen werden. Sind die Zapfen 56 und 57 auf Höhe der Längsschlitze 16 verdreht worden, unterstützt die Feder 38 ein Ausschieben des Bajonetteinsatzes 52 aus der Bajonetthülse 12.

## Patentansprüche

1. Bajonettkupplung (10) zum lösbaren Verbinden zweier Rohrschaftinstrumente oder -instrumententeile, mit einer eine Bajonettführung (14) aufweisenden Bajonetthülse (12), weibliches Teil, an dem einen Instrument bzw. -instrumententeil und einem Bajonetteinsatz (52), männliches Teil, am anderen Instrument bzw. -instrumententeil, und mit einer Verriegelung zum Verriegeln der geschlossenen Bajonettkupplung (10), wobei die Verriegelung ein längs der Bajonetthülse (12) verschiebbares Verriegelungsstück (28) aufweist, das mit dem in die Bajonetthülse (12) eingesetzten Bajonetteinsatz (52) in dessen Endstellung unmittelbar in sperrenden Eingriff kommt und den Bajonetteinsatz (52) gegen Verdrehen sperrt, und das, zum Lösen der Verriegelung, vom Bajonetteinsatz (52) weg bewegbar ist, **dadurch gekennzeichnet, daß** das Verriegelungsstück (28) mit einer äußeren Schiebehülse (26) verbunden ist, die von Hand ergreifbar und verschiebbar ist.

2. Bajonettkupplung nach Anspruch 1, **dadurch gekennzeichnet, daß** das Verriegelungsstück (28) durch die Kraft einer Feder (38) beaufschlagt ist, die das Verriegelungsstück (28) in den sperrenden Eingriff mit dem Bajonetteinsatz (52) drückt.

3. Bajonettkupplung nach Anspruch 1 oder 2, **dadurch gekennzeichnet, daß** das Verriegelungsstück (28) auf der dem Bajonetteinsatz (52) zugewandten Seite eine Ausnehmung (40) aufweist, die, in der Endstellung des Bajonetteinsatzes (52), auf einen Stirnabschnitt (55) des Bajonetteinsatzes (52) aufschiebbar ist, und daß das aufgeschobene Verriegelungsstück (28) ein Drehen des Bajonetteinsatzes (52) verhindert.

4. Bajonettkupplung nach einem der Ansprüche 1 bis 3, **dadurch gekennzeichnet, daß** das Verriegelungsstück (28) eine solche, dem Bajonetteinsatz (52) zugewandte Stirnfläche (44) aufweist, daß der in die Bajonetthülse (12) eingeschobene Bajonetteinsatz (52) das Verriegelungsstück (28) zunächst zurückschiebt und bis in die Endstellung relativ zum Verriegelungsstück (28) verdrehbar ist.

5. Bajonettkupplung nach einem der Ansprüche 1 bis 4, **dadurch gekennzeichnet, daß** das Verriegelungsstück (28) eine mittig durchgehende Bohrung (42) aufweist.

6. Bajonettkupplung nach Anspruch 5, **dadurch gekennzeichnet, daß** alle Bauteile eine durchgehende Bohrung aufweisen.

## Claims

1. Bayonet coupling (10) for detachable joining of two tubular-shaft instruments or instrument parts, with a bayonet sleeve (12), female part, having a bayonet guide (14), on the one instrument or instrument part, and a bayonet insert (52), male part, on the other instrument or instrument part, and with an interlock system to lock the bayonet coupling (10) when closed, wherein the interlock system has an interlock piece (28), displaceable along the bayonet sleeve (12), which comes directly into inhibiting engagement with the bayonet insert (52) inserted into the bayonet sleeve (12), in the insert's terminal position, and inhibits the bayonet insert (52) from rotating, and which can be moved away from the bayonet insert (52) in order to release the interlock system, **characterized in that** the interlock piece (28) is joined to an outer sliding sleeve (26) which can be grasped and displaced manually.

2. Bayonet coupling of claim 1, **characterized in that** the interlock piece (28) is acted upon by the force of a spring (38) which pushes the interlock piece (28) into inhibiting engagement with the bayonet insert (52).

3. Bayonet coupling of claims 1 or 2, **characterized in that** the interlock piece (28) has, on the side facing the bayonet insert (52), a recess (40) which, in the terminal position of the bayonet insert (52), can be slit onto an end section (55) of the bayonet insert (52), and once slit on, the interlock piece (28) inhibits rotation of the bayonet insert (52).

4. Bayonet coupling of anyone of claims 1 through 3, **characterized in that** the interlock piece (28) has an end face (44), facing the bayonet insert (52), such that the bayonet insert (52) pushed into the bayonet sleeve (12) first pushes the interlock piece (28) back and can be rotated relative to the interlock piece (28) into the terminal position.

5. Bayonet coupling of anyone of claims 1 through 4, **characterized in that** the interlock piece (28) has a centered continuous bore (42).

6. Bayonet coupling of claim 5, **characterized in that** all components are equipped with a continuous bore.

## Revendications

1. Accouplement à baïonnette (10) destiné à raccorder de façon détachable deux instruments à tige tubulaire ou deux parties d'instruments à tige tubulaire, comprenant un manchon à baïonnette (12) - partie femelle - présentant un guidage à baïonnette (14), présent sur l'un des instruments ou l'une des parties d'instrument, et un insert à baïonnette (52) - partie mâle - présent sur l'autre instrument ou partie d'instrument, et comprenant un dispositif de verrouillage pour verrouiller l'accouplement à baïonnette (10) fermé, le verrouillage présentant un élément de verrouillage (28) déplaçable le long du manchon à baïonnette (12), qui vient directement coopérer avec et bloquer l'insert à baïonnette (52) inséré dans le manchon à baïonnette (12), dans sa position terminale, et empêche l'insert à baïonnette (52) de tourner et qui est susceptible d'être éloigné de l'insert à baïonnette (52) pour déverrouiller l'accouplement, **caractérisé en ce que** l'élément de verrouillage (28) est relié à un manchon coulissant externe (26) qui peut être saisi et déplacé à la main.

2. Accouplement à baïonnette selon la revendication 1, **caractérisé en ce que** l'élément de verrouillage (28) est sollicité par la force d'un ressort (38) qui contraint l'élément de verrouillage (28) à coopérer avec l'insert à baïonnette (52) et à le bloquer.

3. Accouplement à baïonnette selon la revendication 1 ou 2, **caractérisé en ce que** l'élément de verrouillage (28) présente, sur le côté orienté vers l'insert à baïonnette (52), un évidement (40) qui, dans la position terminale de l'insert à baïonnette (52), est susceptible d'être amené à coulisser sur un tronçon frontal (55) de l'insert à baïonnette (52), et **en ce que** l'élément de verrouillage (28) déplacé empêche une rotation de l'insert à baïonnette (52).

4. Accouplement à baïonnette selon l'une des revendications 1 à 3, **caractérisé en ce que** l'élément de verrouillage (28) présente une surface frontale (44) orientée vers l'insert à baïonnette (52) qui est conformée de telle sorte que l'insert à baïonnette (52) inséré dans le manchon à baïonnette (12) pousse tout d'abord en arrière l'élément de verrouillage (28) et est susceptible d'être tourné jusque dans la position terminale par rapport à l'élément de verrouillage (28).

5. Accouplement à baïonnette selon l'une des revendications 1 à 4, **caractérisé en ce que** l'élément de verrouillage (28) présente un perçage (42) central traversant.

6. Accouplement à baïonnette selon la revendication 5, **caractérisé en ce que** tous les composants présentent un perçage traversant.
